(19) Europäisches Patentamt
European Patent Office
Office européen des brevets

(11) **EP 4 134 109 B1**

(12) **EUROPEAN PATENT SPECIFICATION**

(45) Date of publication and mention
of the grant of the patent:
**01.10.2025 Bulletin 2025/40**

(21) Application number: **21785225.0**

(22) Date of filing: **07.04.2021**

(51) International Patent Classification (IPC):
*A61L 31/14* (2006.01)  *A61L 27/34* (2006.01)
*A61L 27/04* (2006.01)  *A61L 27/30* (2006.01)
*A61L 27/54* (2006.01)  *A61L 27/58* (2006.01)
*A61L 31/02* (2006.01)  *A61L 31/10* (2006.01)
*A61L 31/16* (2006.01)

(52) Cooperative Patent Classification (CPC):
(C-Sets available)
**A61L 31/16; A61L 27/047; A61L 27/34;
A61L 27/54; A61L 27/58; A61L 31/022;
A61L 31/10; A61L 31/148;** A61L 2300/41;
A61L 2300/416; A61L 2300/42; A61L 2420/00
(Cont.)

(86) International application number:
**PCT/CN2021/000059**

(87) International publication number:
**WO 2021/203762 (14.10.2021 Gazette 2021/41)**

(54) **ZINC-CONTAINING MEDICAL INSTRUMENT**

ZINKHALTIGES MEDIZINISCHES INSTRUMENT

INSTRUMENT MÉDICAL CONTENANT DU ZINC

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.04.2020 CN 202010266038**

(43) Date of publication of application:
**15.02.2023 Bulletin 2023/07**

(73) Proprietor: **Biotyx Medical (Shenzhen) Co., Ltd.
Shenzhen, Guangdong 518000 (CN)**

(72) Inventors:
• **ZHANG, Deyuan
Shenzhen, Guangdong 518000 (CN)**
• **QI, Haiping
Shenzhen, Guangdong 518000 (CN)**
• **LIN, Wenjiao
Shenzhen, Guangdong 518000 (CN)**

(74) Representative: **Prinz & Partner mbB
Patent- und Rechtsanwälte
Rundfunkplatz 2
80335 München (DE)**

(56) References cited:
WO-A1-2016/167460      CN-A- 102 961 787
CN-A- 104 857 570      CN-A- 106 474 545
CN-A- 106 806 938      CN-A- 109 688 982
CN-A- 109 954 171      US-A1- 2004 034 409
US-A1- 2005 209 680

(52) Cooperative Patent Classification (CPC): (Cont.)

C-Sets
**A61L 27/34, C08L 67/04;**
**A61L 31/10, C08L 67/04**

**Description**

Technical Field

The present invention relates to the field of interventional medical devices, and more particularly relates to a zinc-containing medical device.

Background Art

This section provides merely background information related to the present invention, which is not necessarily the existing art.

At present, the matrix materials of absorbable implantable medical devices are mainly selected from degradable polymers and corrodible metals. Among the degradable polymers, polylactic acid is the most widely used. The advantage of polylactic acid is that it can completely degrade to degradation products including carbon dioxide and water, but it has insufficient mechanical properties as its disadvantage. The advantages of corrodible metals are easy to process and plastic, and have high mechanical strength. Commonly used corrodible metals in clinic mainly include magnesium and magnesium-based alloys, iron and iron-based alloys, and zinc and zinc-based alloys. WO 2016/167460 A1 discloses a biodegradable stent made of magnesium, coated with a poly-L-lactic acid layer having a thickness of 5-20 microns. CN 104 857 570 A discloses a degradable zinc-based alloy stent comprising a degradable zinc-based material support substrate under the form of a cylindrical mesh, wherein the surface of the support substrate is provided with a degradable polymer eluting coating containing a therapeutic drug. CN 106 474 545 A discloses an absorbable iron-based alloy implantable medical device, comprising an iron-based alloy substrate and a zinc-containing protective body disposed on the surface of the iron-based alloy substrate, further comprising a degradable polymer disposed on the surface of the zinc layer.

From the perspective of clinical application, on the one hand, the device needs to maintain structural integrity and have sufficient mechanical properties from the time of implantation until a diseased part heals and its morphology and functions return to normal. This requires that the rate of early corrosion or the rate of degradation of absorbable implantable medical devices be as slow as possible. On the other hand, the corrosion of a corrodible metal material will generate corrosion products including metal ions. Higher metal ion concentration may be toxic, resulting in biological risk. For example, it has been reported that the cytotoxic (LD50) concentrations of zinc ions to fibroblasts, smooth muscle cells and endothelial cells are 50 $\mu$mol/L, 70 $\mu$mol/L, and 265 $\mu$mol/L, respectively. Therefore, for zinc-containing medical devices, the corrosion rate of zinc must be controlled to maintain sufficient mechanical properties during the repair period and reduce the concentration of zinc ions, thereby further reducing the biological risk.

Summary of the Invention

Based on this, it is necessary to provide a zinc-containing absorbable device that can maintain sufficient mechanical properties during the repair period with low biological risk.

The object of the invention is solved by a zinc-containing medical device according to claim 1.

The zinc-containing medical device comprises a zinc-containing matrix and a polylactic acid coating disposed on the surface of the zinc-containing matrix, wherein the zinc-containing matrix has an outer surface, an inner surface, and a side surface. The polylactic acid coating covers the outer surface, the inner surface, and the side surface. The average thickness of the part located on the outer surface of the polylactic acid coating is $x_{outer}$, the average thickness of the part located on the inner surface of the polylactic acid coating is $x_{inner}$. The average thickness of the part located on the side surface of the polylactic acid coating is $x_{side}$, wherein $x_{inner} \leq x_{outer}$, $x_{inner} \leq x_{side}$, and at least one of $x_{outer}$, $x_{inner}$, and $x_{side}$ that satisfies the following formula:

$$\left(-b + \sqrt{b^2 - 4ac}\right)\Big/ 2a - 2 \leq x \leq \left(-b + \sqrt{b^2 - 4ac}\right)\Big/ 2a + 2$$

,

wherein $x = x_{inner}$, $x_{side}$ or $x_{outer}$, wherein, when the polylactic acid is poly-racemic lactic acid, a=0.0336ln(Mn)-0.1449, b=-0.472ln(Mn)+2.1524, and c=1.1604ln(Mn)-5.7128;

when the polylactic acid is poly-L-lactic acid, a=-0.006ln(Mn)+0.03441, b=0.0648ln(Mn)-0.3662, and c=-0.162ln(Mn)+0.7847; and

Mn is the weight-average molecular weight of the polylactic acid, which is in kDa; and x is in μm.

In one of the embodiments, the polylactic acid is poly-racemic lactic acid with a weight-average molecular weight of 100-300 kDa; and the average thickness of the part located on the outer surface of the polylactic acid coating is 5.2-11.5 μm; the average thickness of the part located on the inner surface of the polylactic acid coating is 5.2-11.5 μm; and the average thickness of the part located on the side surface of the polylactic acid coating is 5.2-11.5 μm.

In one of the embodiments, the polylactic acid is poly-racemic lactic acid with an weight-average molecular weight of 10-100 kDa; and the average thickness of the part located on the outer surface of the polylactic acid coating is 2-9 μm; the average thickness of the part located on the inner surface of the polylactic acid coating is 2-9 μm; and the average thickness of the part located on the side surface of the polylactic acid coating is 2-9 μm.

In one of the embodiments, the polylactic acid is poly-racemic lactic acid with an weight-average molecular weight of 2-10 kDa; and the average thickness of the part located on the outer surface of the polylactic acid coating is 1.5-5.5 μm; the average thickness of the part located on the inner surface of the polylactic acid coating is 1.5-5.5 μm; the average thickness of the part located on the side surface of the polylactic acid coating is 1.5-5.5 μm.

In one of the embodiments, the polylactic acid is poly-L-lactic acid with an weight-average molecular weight of 200-300 kDa; and the average thickness of the part located on the outer surface of the polylactic acid coating is 9-22 μm; the average thickness of the part located on the inner surface of the polylactic acid coating is 9-22 μm; and the average thickness of the part located on the side surface of the polylactic acid coating is 9-22 μm.

In one of the embodiments, the polylactic acid is poly-L-lactic acid with an weight-average molecular weight of 50-200 kDa; and the average thickness of the part located on the outer surface of the polylactic acid coating is 7-13 μm; the average thickness of the part located on the inner surface, of the polylactic acid coating is 7-13 μm; and the average thickness of the part located on the side surface of the polylactic acid coating is 7-13 μm.

According to the invention, the material of the zinc-containing matrix is pure zinc or zinc alloy; or, the zinc-containing matrix includes a body and a zinc-containing layer attached to the body; and the material of the zinc-containing layer is pure zinc or zinc alloy.

According to the invention, when the material of the zinc-containing matrix is pure zinc or zinc alloy, the mass percentage of zinc in the zinc alloy is 50%-99.99%;

when the zinc-containing matrix includes the body and the zinc-containing layer attached to the body, and the material of the zinc-containing layer is zinc alloy, the mass percentage of zinc in the zinc alloy is 50%-99.99%.

According to the invention, the zinc-containing layer covers all surfaces of the body.

In one of the embodiments, the polylactic acid coating contains an active drug.

It has been proved by experiment that when the weight-average molecular weight Mn (kDa) of the polylactic acid in the polylactic acid coating and the thickness x (μm) of the polylactic acid coating satisfy the following formula

$$\left.\left(-b+\sqrt{b^2-4ac}\right)\middle/2a - 2 \le x \le \left(-b+\sqrt{b^2-4ac}\right)\middle/2a + 2\right.,$$

and when the polylactic acid is poly-racemic lactic acid, a=0.0336ln(Mn)-0.1449, b=-0.472ln(Mn)+2.1524, and c=1.1604ln(Mn)-5.7128; when the polylactic acid is poly-L-lactic acid, a=-0.006ln(Mn)+0.03441, b=0.0648ln(Mn)-0.3662, and c=-0.162ln(Mn)+0.7847, the corrosion rate of zinc in the zinc-containing matrix is relatively small, to prevent fast corrosion of the zinc-containing matrix, and can maintained sufficient mechanical properties during the repair period. Furthermore, the lower corrosion rate of the zinc in the zinc-containing matrix can avoid an extremely high cumulative concentration of zinc ions, which is conducive to reducing biological risk.

Brief Description of the Drawings

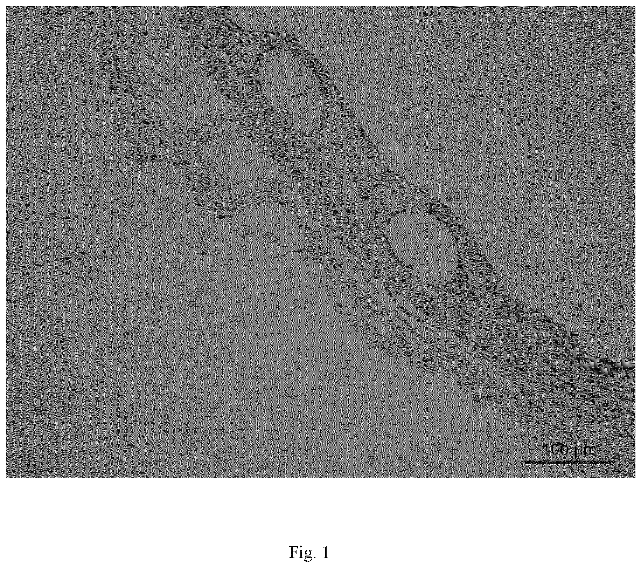

Fig. 1 is a pathological section diagram of the tissue around the iron-based stent after the iron-based stent of Example 5 was implanted into the rabbit iliac artery for 1 month.

Detailed Description of the Invention

In order to make the foregoing objectives, features and advantages of the present invention more obvious and understandable, the specific embodiments of the present invention will be described in detail below with reference to the accompanying drawings. In the following description, many specific details are described to provide a thorough understanding of the present invention. Therefore, the present invention is not limited by the specific embodiments disclosed below.

Unless otherwise defined, all technical and scientific terms used herein have the same meanings as commonly understood by those skilled in the art to which the present invention belongs. The terms used herein in the description of the present invention are merely for the purpose of describing specific embodiments and are not intended to limit the present invention.

The zinc-containing medical device includes a zinc-containing matrix and a polylactic acid coating arranged on the zinc-containing matrix. Among that, the zinc-containing matrix is an absorbable matrix. The polylactic acid coating at least covers a partial surface of the zinc-containing matrix.

According to the invention, the material of the zinc-containing matrix is pure zinc or zinc alloy, that is, the zinc-containing matrix is made of pure zinc or zinc alloy. For example, the zinc-containing matrix is a hollow lumen structure made of pure zinc or a zinc alloy. Among them, the zinc alloy is a bioabsorbable alloy.

In one embodiment, the alloying element in the zinc alloy is at least one of C, N, O, S, P, Ce, Mn, Ca, Cu, Pd, Si, W, Ti, Co, Cr, Cu, and Re. It should be noted that the alloying elements in the zinc alloy are non-toxic and harmless to living organisms. Alternatively, the content of the alloying elements is low enough not to cause toxic effects on the organisms.

The mass percentage of zinc in the zinc alloy is 50%-99.99%.

In another embodiment, the zinc-containing matrix includes a body and a zinc-containing layer attached to the body, and the material of the zinc-containing layer is pure zinc or zinc alloy. The material of the body is an absorbable metal or absorbable polymer. For example, the absorbable metal is pure iron or iron-based alloy. The zinc-containing layer covers all surfaces of the body.

When the material of the zinc-containing layer is the zinc alloy, the alloying element in the zinc alloy is at least one of C, N, O, S, P, Ce, Mn, Ca, Cu, Pd, Si, W, Ti, Co, Cr, Cu, and Re. It should be noted that the alloying element in the zinc alloy is non-toxic and harmless to living organisms. Alternatively, the content of the alloying element is low enough not to cause toxic effects on living organisms. In one embodiment, the mass percentage of zinc in the zinc alloy is 50%-99.99%.

Whether the zinc-containing matrix is a matrix made of pure zinc or zinc alloy or the zinc-containing matrix includes a body and a zinc-containing layer attached to the body, in one embodiment, the zinc-containing matrix has an outer surface, an inner surface, and a side surface. Wherein, when the zinc-containing medical device is implanted into the body, the inner surface is the surface in direct contact with body fluids (e.g. blood), the outer surface is the surface in direct contact with the tissue walls (e.g. vascular walls), and the side surface is connected to the inner surface and outer surface.

According to the invention, the polylactic acid coating covers the outer surface, the inner surface, and the side surface of the zinc-containing matrix.

When the zinc-containing medical device is implanted into the body, zinc corrosion can generate zinc phosphate, at the same time, the polylactic acid is degraded to generate carboxyl and hydroxyl. Zinc phosphate can react with carboxyl and hydroxyl to form complexes, thereby hindering further corrosion of the zinc. Thus, the zinc corrosion rate of the zinc-containing medical device that includes a polylactic acid coating is less than the zinc corrosion rate of the zinc-containing medical device that does not include a polylactic acid coating. Meanwhile, hydrogen ions generated by the degradation of the polylactic acid will accelerate the corrosion of metals. That is, the polylactic acid coating has both inhibition effects and promotion effects on the corrosion rate of the zinc in the zinc-containing matrix. The release rate and cumulative concentration of PLA degradation products will affect the corrosion inhibition and promotion effects of the polylactic acid coating on the zinc corrosion. The properties of the polylactic acid itself in the polylactic acid coating are an important factor affecting the degradation rate of the polylactic acid. The thickness of the polylactic acid coating affects the total amount of the degradation products of the polylactic acid.

The thickness of the polylactic acid coating is x, which is in $\mu$m, and satisfies the following formula:

$$\left(-b + \sqrt{b^2 - 4ac}\right)\Big/2a - 2 \leq x \leq \left(-b + \sqrt{b^2 - 4ac}\right)\Big/2a + 2,$$

wherein, when the polylactic acid is poly-racemic lactic acid, a=0.0336ln(Mn)-0.1449, b=-0.472ln(Mn)+2.1524, and c=1.1604ln(Mn)-5.7128; and

when the polylactic acid is poly-L-lactic acid, a=-0.006ln(Mn)+0.03441, b=0.0648ln(Mn)-0.3662, and c=-0.162ln(Mn)+0.7847.

Among them, Mn is the weight-average molecular weight of the polylactic acid, which is in kDa. That is, when calculating a, b, and c, the numerical value of Mn is put into the formulas according to the numerical value in kDa for calculation. The calculated numerical values are directly used in the above formula or relational expression. When the thickness x of the polylactic acid coating is put into the above formula or relational expression, the corresponding numerical value in $\mu$m of the thickness of the polylactic acid coating is put into the above relational expression for comparison.

The above thickness x of the polylactic acid coating refers to the average thickness $x_{outer}$ of the part located on the outer surface of the polylactic acid coating; the average thickness $x_{inner}$ of the part located on the inner surface of the polylactic acid coating; or the average thickness $x_{side}$ of the part located on the side surface of the polylactic acid coating. That is, at least one of $x_{outer}$, $x_{inner}$, and $x_{side}$ satisfies the above formula.

According to the invention, $x_{inner} \leq x_{outer}$, $x_{inner} \leq x_{side}$, and at least one of $x_{outer}$, $x_{inner}$, and $x_{side}$ satisfies the following formula:

$$\left(-b + \sqrt{b^2 - 4ac}\right)\big/_{2a} - 2 \leq x \leq \left(-b + \sqrt{b^2 - 4ac}\right)\big/_{2a} + 2 \, ,$$

where, $x = x_{inner}$, $x_{side}$ or $x_{outer}$.

In one embodiment, $x_{outer}$, $x_{inner}$, and $x_{side}$ all satisfy the above formula.

It has been proved by experiments that when the thickness of the polylactic acid coating, the type of the polylactic acid, and the molecular weight of the polylactic acid satisfy the above relationship, the corrosion inhibition effect is dominant among the effects of the polylactic acid coating on the corrosion rate of the zinc in the zinc-containing matrix, so that the corrosion rate of the zinc is relatively small.

By arranging the polylactic acid coating with the thickness of x μm on the zinc-containing matrix, and selecting the suitable polylactic acid, the corrosion rate of the zinc in the zinc-containing matrix is relatively small. When the zinc-containing matrix is a matrix made of pure zinc or zinc alloy, the corrosion rate of the zinc is relatively small, that is, the corrosion rate of the zinc-containing matrix itself is relatively small, so as to avoid fast corrosion of the zinc-containing matrix and quick loss of mechanical properties, which is thus conducive to maintaining sufficient mechanical properties during the repair period. When the zinc-containing matrix includes a body and a zinc-containing layer attached to the body, and the material of the zinc-containing layer is pure zinc or zinc alloy, the zinc-containing layer is coated on the surface of the body to physically isolate the body and body fluids. The corrosion rate of the zinc-containing layer is relatively small, which is conducive to delaying the protection period of the body, thereby maintaining sufficient mechanical properties during the repair period.

Furthermore, due to the small corrosion rate of zinc, the cumulative concentration of corrosion products of zinc in tissues is relatively low, which reduces the biological risk.

It should be noted that when the thickness x of the polylactic acid coating satisfies the above formula, the corrosion rate of the zinc in the zinc-containing matrix is relatively small, which means that comparison is carried out under the same premise of other conditions, such as same zinc-containing matrix, same polylactic acid material, and same distribution of the polylactic acid coating. Alternatively, under the premise of the same zinc-containing matrix, the corrosion rate of zinc in a zinc-containing medical device that includes the above polylactic acid coating is smaller than that of zinc in a device which does not include the polylactic acid coating.

In one embodiment, the polylactic acid is poly-racemic lactic acid with the weight-average molecular weight of 100-300 kDa; the average thickness of the part located on the outer surface of the polylactic acid coating is 5.2-11.5 μm; and/or, the average thickness of the part located on the inner surface of the polylactic acid coating is 5.2-11.5 μm; and/or, the average thickness of the part located on the side surface of the polylactic acid coating is 5.2-11.5 μm. In one embodiment, the average thicknesses of all the surfaces of the polylactic acid coating are 5.2-11.5 μm.

In one embodiment, the polylactic acid is poly-racemic lactic acid with the weight-average molecular weight of 10-100 kDa; the average thickness of the part located on the outer surface of the polylactic acid coating is 2-9 μm; and/or, the average thickness of the part located on the inner surface of the polylactic acid coating is 2-9 μm; and/or, the average thickness of the part located on the side surface of the polylactic acid coating is 2-9 μm. In one embodiment, the average thicknesses of all the surfaces of the polylactic acid coating are 2-9 μm.

In one embodiment, the polylactic acid is poly-racemic lactic acid with the weight-average molecular weight of 2-10 kDa; the average thickness of the part located on the outer surface of the polylactic acid coating is 1.5-5.5 μm; and/or, the average thickness of the part located on the inner surface of the polylactic acid coating is 1.5-5.5 μm; and/or, the average thickness of the part located on the side surface of the polylactic acid coating is 1.5-5.5 μm. In one embodiment, the average thicknesses of all the surfaces of the polylactic acid coating are 1.5-5.5 μm.

In one embodiment, the polylactic acid is poly-racemic lactic acid with the weight-average molecular weight of 200 kDa; the average thickness of the part located on the outer surface of the polylactic acid coating is 9.1 μm; and/or, the average thickness of the part located on the inner surface of the polylactic acid coating is 9.1 μm; and/or, the average thickness of the part located on the side surface of the polylactic acid coating is 9.1 μm. In one embodiment, the average thicknesses of all the surfaces of the polylactic acid coating are 9.1 μm.

In one embodiment, the polylactic acid is poly-racemic lactic acid with the weight-average molecular weight of 50 kDa; the average thickness of the part located on the outer surface of the polylactic acid coating is 4.9 μm; and/or, the average thickness of the part located on the inner surface of the polylactic acid coating is 4.9 μm; and/or, the average thickness of the part located on the side surface of the polylactic acid coating is 4.9 μm. Alternatively, in one embodiment, the average thicknesses of all the surfaces of the polylactic acid coating are 4.9 μm.

In one embodiment, the polylactic acid is poly-racemic lactic acid with an weight-average molecular weight of 5 kDa; the average thickness of the part located on the outer surface of the polylactic acid coating is 3.6 $\mu$m; and/or, the average thickness of the part located on the inner surface of the polylactic acid coating is 3.6 $\mu$m; and/or, the average thickness of the part located on the side surface of the polylactic acid coating is 3.6 $\mu$m. In one embodiment, the average thicknesses of all the surfaces of the polylactic acid coating are 3.6 $\mu$m.

In one embodiment, the polylactic acid is poly-L-lactic acid with the weight-average molecular weight of 200-300 kDa; the average thickness of the part located on the outer surface of the polylactic acid coating is 9-22 $\mu$m; or the average thickness of the part located on the inner surface of the polylactic acid coating is 9-22 $\mu$m; or the average thickness of the part located on the side surface of the polylactic acid coating is 9-22 $\mu$m. In one embodiment, the average thicknesses of all the surfaces of the polylactic acid coating are 9-22 $\mu$m.

In one embodiment, the polylactic acid is poly-L-lactic acid with the weight-average molecular weight of 50-200 kDa; the average thickness of the part located on the outer surface of the polylactic acid coating is 7-13 $\mu$m; or the average thickness of the part located on the inner surface of the polylactic acid coating is 7-13 $\mu$m; or the average thickness of the part located on the side surface of the polylactic acid coating is 7-13 $\mu$m. In one embodiment, the average thicknesses of all the surfaces of the polylactic acid coating are 7-13 $\mu$m.

In one embodiment, the polylactic acid is poly-L-lactic acid with the weight-average molecular weight of 300 kDa; the average thickness of the part located on the outer surface of the polylactic acid coating is 19.7 $\mu$m; or the average thickness of the part located on the inner surface of the polylactic acid coating is 19.7 $\mu$m; or the average thickness of the part located on the side surface of the polylactic acid coating is 19.7 $\mu$m. In one embodiment, the average thicknesses of all the surfaces of the polylactic acid coating are 19.7 $\mu$m.

In one embodiment, the polylactic acid is poly-L-lactic acid with the weight-average molecular weight of 100 kDa; the average thickness of the part located on the outer surface of the polylactic acid coating is 9.4 $\mu$m; or the average thickness of the part located on the inner surface of the polylactic acid coating is 9.4 $\mu$m; or the average thickness of the part located on the side surface of the polylactic acid coating is 9.4 $\mu$m. In one embodiment, the average thicknesses of all the surfaces of the polylactic acid coating are 9.4 $\mu$m.

In one embodiment, the polylactic acid coating contains an active drug. In one embodiment, the active drug is selected from at least one of the drugs that inhibit vascular proliferation, anti-platelet drugs, anti-thrombotic drugs, anti-inflammatory drugs, and anti-allergic drugs. The drug that inhibits vascular proliferation is at least one of paclitaxel, rapamycin, and derivatives thereof. The anti-platelet drug may be cilostazol. The anti-thrombotic drug may be heparin. The anti-inflammatory drug may be dexamethasone. The anti-allergic drug is selected from at least one of calcium gluconate, chlorpheniramine, and cortisone. When the polylactic acid coating contains an active drug, that is, when polylactic acid is mixed with the active drug to form a mixed coating, the thickness of the polylactic acid coating can be converted from the thickness of the mixed coating according to the mass ratio of the polylactic acid to the drug: the thickness (x) of the polylactic acid coating=the thickness of the mixed coating $\times$ the mass percentage of the polylactic acid. In one embodiment, the active drug may be distributed in at least one of the outer surfaces, the inner surface, and the side surface of the zinc-containing matrix.

In one embodiment, the polylactic acid is poly-racemic lactic acid with the weight-average molecular weight of 5 kDa. The average thicknesses of the parts located on the inner surface and the side surface of the polylactic acid coating are 0, that is, the polylactic acid coating does not cover the inner surface and the side surface. The part located on the outer surface contains sirolimus; the average thickness of the polylactic acid-sirolimus coating is 5.4 $\mu$m; and the average thickness of the part located on the outer surface of the polylactic acid coating is converted to be 3.6 $\mu$m. The mass ratio of the polylactic acid to the sirolimus is 2: 1.

In one embodiment, the polylactic acid is poly-racemic lactic acid with the weight-average molecular weight of 200 kDa. The average thickness of the part located on the inner surface of the polylactic acid coating is 8 microns; the parts located on the side surface and the outer surface of the polylactic acid coating both contain sirolimus, that is, the polylactic acid coating is the polylactic acid-sirolimus coating in which the mass ratio of the polylactic acid to the sirolimus is 6: 1. The average thicknesses of the parts located on the side surface and the outer surface of the poly-racemic lactic acid-sirolimus coating are 10.6 $\mu$m, and the average thickness of the polylactic acid coating is converted to be 9.1 $\mu$m.

The above zinc-containing medical device may be a vascular stent, a non-vascular endoluminal stent, an occluder, an orthopedic implant, a dental implant, a respiratory implant, a gynecological implant, an andrology implant, a suture, or a bolt. The non-vascular endoluminal stent may be a tracheal stent, an esophageal stent, a urethral stent, an intestinal stent, or a biliary stent. The orthopedic implant may be a fixation screw, a fixation rivet, or a bone plate. Of course, other medical devices that need to achieve degradable and absorption can be used as the medical devices of this embodiment.

The above-mentioned zinc-containing medical devices are further described below through specific examples.

The test methods of the following examples are as follows:

    1. Determination of the weight-average molecular weight of polylactic acid

The weight-average molecular weight is tested using a GPC-multiangle laser light scattering apparatus combined with a molecular weight test system (Wyatt, USA). The test system includes a liquid phase pump and a sample injector (Agilent, USA), an Agilent PLMIXED-C GPC column (size: 7.5×300 mm, 5 μm) (Agilent, USA), and a multi-angle laser light scattering apparatus and a differential detector (Wyatt, USA). Detection conditions are as follows: Mobile phase: tetrahydrofuran; pump flow rate: 1 mL/min; injection volume: 100 μL; laser wavelength: 663.9 nm; and test temperature: 35°C.

2. Determination of the thickness of the polylactic acid coating

The thickness is measured using a scanning electron microscope: a sample requiring coating thickness test is fixed on a sample holder; the sample holder is then put in metal spraying equipment JFC-1600 for platinum spraying; after being sprayed once, the sample is rotated 180 degrees for being sprayed one more time to ensure all positions are sprayed; the sample sprayed with a metal on the surface is added to a Buehler room temperature resin curing agent mixed reagent prepared in a ratio of 5: 1, and is made to stand for more than 8 hours before the sample can be released from a sealing shell; the sealed sample is divided into 3 segments equally; each segment is ground and polished with a semi-automatic grinding and polishing machine according to a sample polishing and grinding procedure, and a cross section of the sample to be measured shall be polished until there are no wear marks; the polished sample is fixed on an object stage of the scanning electron microscope, and the entire object stage is placed in the metal spraying equipment JFC-1600 for metal spraying for 20 s; and the metal-sprayed sample is placed in a scanning electron microscope JSM-6510 for thickness measurement. The thickness of the inner surface coating, the thickness of the side surface coating, and the thickness of the outer surface coating are respectively measured for each cross-section. If there are multiple rods in each cross-section, at least three rods are randomly selected to measure the thicknesses of the inner surface coatings, the thicknesses of the side surface coatings, and the thicknesses of the outer surface coatings of the rods. An average value of all the measured thicknesses of the inner surface coatings is calculated to be the average thickness of the inner surface coating of the device; an average value of all the measured thicknesses of the side surface coatings is calculated to be the average thickness of the side surface coating of the device, and the average value of all the measured thicknesses of the outer surface coatings is calculated to be the average thickness of the outer surface coating of the device.

3. Determination of the corrosion rate of zinc in zinc-containing medical devices

The corrosion rate is detected by a weight loss method: a zinc-containing medical device is implanted into the body of a rabbit; and the rabbit is killed at a predetermined observation time point such as 1 month and 3 months, and the zinc-containing medical device is taken out. After the tissue on the zinc-containing medical device is carefully removed as much as possible, the zinc-containing medical device is then soaked in a saturated glycine solution and ultrasonically cleaned. After 1 min, the zinc-containing medical device is taken out of the glycine solution and immediately washed with water for 10 s. The cleaned zinc-containing medical device is soaked in 1 mol/L sodium hydroxide solution for more than 24 h to completely dissolve zinc. The concentration of zinc in the sodium hydroxide solution is detected by atomic absorption spectrometry (AAS). The corrosion rate of zinc in the zinc-containing medical device can be obtained by calculation.

Example 1

A zinc-based stent with a specification of 3.0 mm × 8 mm included a stent matrix and a polylactic acid coating completely covering the surfaces of the stent matrix. The material of the stent matrix was pure zinc, and the stent matrix had a mass of 5 mg. Polylactic acid in the polylactic acid coating was poly-racemic lactic acid with a molecular weight of 200 kDa. The average thickness of the polylactic acid coating on an outer surface, the average thickness of the polylactic acid coating on a side surface, and the average thickness of the polylactic acid coating on an inner surface were all 9.1 μm. The polylactic acid coating was prepared by the spraying method.

Comparative Example 1-1

A zinc-based stent with a specification of 3.0 mm × 8 mm included a stent matrix and a polylactic acid coating completely covering surfaces of the stent matrix. The material of the stent matrix was pure zinc, and the stent matrix had a mass of 5 mg. Polylactic acid in the polylactic acid coating was poly-racemic lactic acid with a molecular weight of 200 kDa. The average thickness of the polylactic acid coating on an outer surface, the average thickness of the polylactic acid coating on a side surface, and the average thickness of the polylactic acid coating on an inner surface were all 6 μm. The polylactic acid coating was prepared by the spraying method.

Comparative Example 1-2

A zinc-based stent with a specification of 3.0 mm × 8 mm included a stent matrix and a polylactic acid coating completely covering surfaces of the stent matrix. The material of the stent matrix was pure zinc, and the stent matrix had a mass of 5 mg. Polylactic acid in the polylactic acid coating was poly-racemic lactic acid with the molecular weight of 200 kDa. The average thickness of the polylactic acid coating on an outer surface, the average thickness of the polylactic acid coating on a side surface, and the average thickness of the polylactic acid coating on an inner surface were all 16 μm. The polylactic acid coating was prepared by the spraying method.

The zinc-based stents of Example 1, Comparative Example 1-1, and Comparative Example 1-2 were respectively implanted into the iliac arteries of three rabbits and were taken out after 6 months. The zinc corrosion rates were measured to be 12%, 20%, and 18%, respectively. The corrosion rate of zinc in the zinc-based stent of Example 1 is the smallest.

Example 2

A zinc-based stent with a specification of 3.0 mm × 8 mm included a stent matrix and a polylactic acid coating completely covering surfaces of the stent matrix. The material of the stent matrix was pure zinc, and the stent matrix had a mass of 5 mg. Polylactic acid in the polylactic acid coating was poly-racemic lactic acid with a molecular weight of 50 kDa. The average thickness of the polylactic acid coating on an outer surface was 6 μm; the average thickness of the polylactic acid coating on a side surface was 5.5 μm; and the average thickness of the polylactic acid coating on an inner surface was 4.9 μm. The polylactic acid coating was prepared by the spraying method.

Example 2-1

A zinc-based stent with a specification of 3.0 mm × 8 mm included a stent matrix and a polylactic acid coating completely covering surfaces of the stent matrix. the material of the stent matrix was pure zinc, and the stent matrix had a mass of 5 mg. Polylactic acid in the polylactic acid coating was poly-racemic lactic acid with a molecular weight of 50 kDa. The average thickness of the polylactic acid coating on an outer surface was 6 μm; the average thickness of the polylactic acid coating on a side surface was 5.5 μm; and the average thickness of the polylactic acid coating on an inner surface was 2.5 μm. The polylactic acid coating was prepared by the spraying method.

Comparative Example 2-2

A zinc-based stent with a specification of 3.0 mm × 8 mm included a stent matrix and a polylactic acid coating completely covering surfaces of the stent matrix. The material of the stent matrix was pure zinc, and the stent matrix had a mass of 5 mg. Polylactic acid in the polylactic acid coating was poly-racemic lactic acid with a molecular weight of 50 kDa. The average thickness of the polylactic acid coating on an outer surface was 6 μm; the average thickness of the polylactic acid coating on a side surface was 5.5 μm; and the average thickness of the polylactic acid coating on an inner surface was 8 μm. The polylactic acid coating was prepared by the spraying method.

The zinc-based stents of Example 2, Example 2-1, and Example 2-2 were respectively implanted into the iliac arteries of three rabbits and were taken out after 6 months. The zinc corrosion rates were measured to be 18%, 28%, and 24%, respectively. The corrosion rate of zinc in the zinc-based stent of Example 2 is the smallest.

Example 3

A zinc-based stent with a specification of 3.0 mm × 8 mm included a stent matrix and a polylactic acid-sirolimus coating only covering an outer surface of the stent matrix. The material of the stent matrix was pure zinc, and the stent matrix had a mass of 5 mg. Polylactic acid in the polylactic acid coating was poly-racemic lactic acid with a molecular weight of 5 kDa. The mass ratio of the poly-racemic lactic acid to the sirolimus was 2: 1. The average thickness of the polylactic acid-sirolimus coating was 5.4 μm, and the average thickness of the polylactic acid coating was converted to be 3.6 μm. The polylactic acid-sirolimus coating was prepared by the 3D printing method.

Comparative Example 3-1

A zinc-based stent with a specification of 3.0 mm × 8 mm included a stent matrix. The material of the stent matrix was pure zinc, and the stent matrix had a mass of 5 mg and is basically provided with no polylactic acid coating.

Comparative Example 3-2

A zinc-based stent with a specification of 3.0 mm × 8 mm included a stent matrix and a polylactic acid-sirolimus coating only covering an outer surface of the stent matrix. The material of the stent matrix was pure zinc, and the stent matrix had a mass of 5 mg. Polylactic acid in the polylactic acid coating was poly-racemic lactic acid with a molecular weight of 5 kDa. The mass ratio of the poly-racemic lactic acid to the sirolimus was 2: 1. The average thickness of the polylactic acid-sirolimus coating was 12 $\mu$m, and the average thickness of the polylactic acid coating was converted to be 8 $\mu$m. The polylactic acid-sirolimus coating was prepared by the spraying method.

The zinc-based stents of Example 3, Comparative Example 3-1, and Comparative Example 3-2 were respectively implanted into the iliac arteries of three rabbits and were taken out after 3 months. The zinc corrosion rates were measured to be 11%, 17%, and 13%, respectively. The corrosion rate of zinc in the zinc-based stent of Example 3 is the smallest.

Example 4

A zinc-based stent with a specification of 3.0 mm × 8 mm included a stent matrix and a polylactic acid coating completely covering the surfaces of the stent matrix. The material of the stent matrix was a zinc alloy, and the stent matrix had a mass of 5 mg. Polylactic acid in the polylactic acid coating was poly-l-lactic acid with a molecular weight of 300 kDa. The average thickness of the polylactic acid coating on an outer surface, the average thickness of the polylactic acid coating on a side surface, and the average thickness of the polylactic acid coating on an inner surface were all 19.6 $\mu$m. The polylactic acid coating was prepared by the spraying method.

Comparative Example 4-1

A zinc-based stent with a specification of 3.0 mm × 8 mm included a stent matrix and a polylactic acid coating completely covering the surfaces of the stent matrix. The material of the stent matrix was pure zinc, and the stent matrix had a mass of 5 mg. Polylactic acid in the polylactic acid coating was poly-l-lactic acid with a molecular weight of 300 kDa. The average thickness of the polylactic acid coating on an outer surface, the average thickness of the polylactic acid coating on a side surface, and the average thickness of the polylactic acid coating on an inner surface were all 10 $\mu$m. The polylactic acid coating was prepared by the spraying method.

Comparative Example 4-2

A zinc-based stent with a specification of 3.0 mm × 8 mm included a stent matrix and a polylactic acid coating completely covering the surfaces of the stent matrix. The material of the stent matrix was pure zinc, and the stent matrix had a mass of 5 mg. Polylactic acid in the polylactic acid coating was poly-l-lactic acid with a molecular weight of 300 kDa. The average thickness of the polylactic acid coating on an outer surface, the average thickness of the polylactic acid coating on a side surface, and the average thickness of the polylactic acid coating on an inner surface were all 30 $\mu$m. The polylactic acid coating was prepared by the spraying method.

The zinc-based stents of Example 4, Comparative Example 4-1, and Comparative Example 4-2 were respectively implanted into the iliac arteries of three rabbits and were taken out after 6 months. The zinc corrosion rates were measured to be 21%, 29%, and 27%, respectively. The corrosion rate of zinc in the zinc-based stent of Example 4 is the smallest.

Example 5

An iron-based stent with a specification of 3.0 mm × 8 mm included a stent body made of an iron-based alloy, a pure zinc layer completely covering the surface of the stent body, and a polylactic acid-sirolimus coating covering the surface of the pure zinc layer. The mass of iron in the stent body was 4 mg; the zinc layer had a mass of 250 $\mu$g; polylactic acid in the coating was poly-racemic lactic acid with a molecular weight of 200 kDa. The mass ratio of the poly-racemic lactic acid-sirolimus coating on an inner surface was 10: 1. The thickness of the poly-racemic lactic acid-sirolimus coating on the inner surface was 5.5 $\mu$m, and the thickness of the polylactic acid coating was converted to be 5 $\mu$m. Mass ratios of the poly-racemic lactic acid-sirolimus coatings on a side surface and an outer surface were 6: 1. Thicknesses of the poly-racemic lactic acid-sirolimus coatings on the side surface and the outer surface were 10.6 $\mu$m, and the thickness of the polylactic acid coating was converted to be 9.1 $\mu$m. The polylactic acid coating and the polylactic acid-sirolimus coating were prepared by the spraying method.

Comparative Example 5-1

An iron-based stent with a specification of 3.0 mm × 8 mm included a stent body made of an iron-based alloy, a pure zinc layer completely covering the surface of the stent body, and a polylactic acid-sirolimus coating covering the surface of the pure zinc layer. The mass of iron in the stent body was 4 mg; the zinc layer had a mass of 250 μg; polylactic acid in the coating was poly-racemic lactic acid with the molecular weight of 200 kDa. The mass ratio of the poly-racemic lactic acid-sirolimus coating on an inner surface was 10: 1. The thickness of the poly-racemic lactic acid-sirolimus coating on the inner surface was 5.5 μm, and the thickness of the polylactic acid coating was converted to be 5 μm. Mass ratios of the poly-racemic lactic acid-sirolimus coatings on a side surface and an outer surface were 6: 1. Thicknesses of the poly-racemic lactic acid-sirolimus coatings on the side surface and the outer surface were 7 μm, and the thickness of the polylactic acid coating was converted to be 6 μm. The polylactic acid coating and the polylactic acid-sirolimus coating were prepared by the spraying method.

Comparative Example 5-2

An iron-based stent with a specification of 3.0 mm × 8 mm included a stent body made of an iron-based alloy, a pure zinc layer completely covering the surface of the stent body, and a polylactic acid-sirolimus coating covering the surface of the pure zinc layer. The mass of iron in the stent body was 4 mg; the zinc layer had a mass of 250 μg; polylactic acid in the coating was poly-racemic lactic acid with a molecular weight of 200 kDa. The mass ratio of the poly-racemic lactic acid-sirolimus coating on an inner surface was 10: 1. The thickness of the poly-racemic lactic acid-sirolimus coating on the inner surface was 5.5 μm, and the thickness of the polylactic acid coating was converted to be 5 μm. Mass ratios of the poly-racemic lactic acid-sirolimus coatings on a side surface and an outer surface were 6: 1. Thicknesses of the poly-racemic lactic acid-sirolimus coatings on the side surface and the outer surface were 17.5 μm, and the thickness of the polylactic acid coating was converted to be 15 μm. The polylactic acid coating and the polylactic acid-sirolimus coating were prepared by the spraying method.

The iron-based stents with Example 5, Comparative Example 5-1, and Comparative Example 5-2 were respectively implanted into the iliac arteries of three rabbits and were taken out after 1 month. The zinc corrosion rates were measured to be 30%, 56%, and 44%, respectively. The corrosion rate of zinc in the iron-based stent of Example 5 was the smallest. It can be seen from the pathological section shown in Fig. 1 that the tissue around the iron-based stent of Example 5 is infiltrated by a few of inflammatory cells, and there is no obvious abnormal change such as tissue necrosis.

Example 6

An iron-based stent with a specification of 3.0 mm × 8 mm included a stent body made of an iron-based alloy, a pure zinc layer completely covering the surface of the stent body, a polylactic acid coating covering an inner surface of the pure zinc layer, and a polylactic acid-sirolimus coating covering a side surface and an outer surface of the pure zinc layer. The mass of iron in the stent body was 4 mg; the zinc layer had a mass of 250 μg; the polylactic acid in the coating was poly-racemic lactic acid with a molecular weight of 200 kDa. The average thickness of the poly-racemic lactic acid coating on the inner surface was 6 μm. Mass ratios of the poly-racemic lactic acid to the sirolimus on the side surface and the outer surface were 3: 1. Thicknesses of the poly-racemic lactic acid-sirolimus coatings on the side surface and the outer surface were 14.7 μm, and the thickness of the polylactic acid coating was converted to be 11 μm.

Comparative Example 6-1

An iron-based stent with a specification of 3.0 mm × 8 mm included a stent body made of an iron-based alloy, a pure zinc layer completely covering the surface of the stent body, a polylactic acid coating covering an inner surface of the pure zinc layer, and a polylactic acid-sirolimus coating covering a side surface and an outer surface of the pure zinc layer. The mass of iron in the stent body was 4 mg; the zinc layer had a mass of 250 μg; the polylactic acid in the coating was poly-racemic lactic acid with a molecular weight of 200 kDa. The average thickness of the poly-racemic lactic acid coating on the inner surface was 6 μm. Mass ratios of the poly-racemic lactic acid to the sirolimus on the side surface and the outer surface were 3: 1. Thicknesses of the poly-racemic lactic acid-sirolimus coatings on the side surface and the outer surface were 8 μm, and the thickness of the polylactic acid coating was converted to be 6 μm. The polylactic acid-sirolimus coating was prepared by the spraying method.

Comparative Example 6-2

An iron-based stent with a specification of 3.0 mm × 8 mm included a stent body made of an iron-based alloy, a pure zinc layer completely covering the surface of the stent body, a polylactic acid coating covering an inner surface of the pure zinc layer, and a polylactic acid-sirolimus coating covering a side surface and an outer surface of the pure zinc layer. The mass of iron in the stent body was 4 mg; the zinc layer had a mass of 250 μg; the polylactic acid in the coating was poly-racemic lactic acid with a molecular weight of 200 kDa. The average thickness of the poly-racemic lactic acid coating on the inner surface was 6 μm. Mass ratios of the poly-racemic lactic acid to the sirolimus on the side surface and the outer surface were 3: 1. Thicknesses of the poly-racemic lactic acid-sirolimus coatings on the side surface and the outer surface were 21 μm, and the thickness of the polylactic acid coating was converted to be 15.8 μm. The polylactic acid coating and the polylactic acid-sirolimus coating were prepared by the spraying method.

The iron-based stents of Example 6, Comparative Example 6-1, and Comparative Example 6-2 were respectively implanted into the iliac arteries of three rabbits and were taken out after 1 month. The zinc corrosion rates were measured to be 33%, 54%, and 49%, respectively. The corrosion rate of zinc in the iron-based stent of Example 6 is the smallest.

Example 7

An iron-based stent with a specification of 3.0 mm × 8 mm included a stent body made of an iron-based alloy, a pure zinc layer completely covering the surface of the stent body, a polylactic acid coating covering an inner surface and a side surface of the pure zinc layer, and a polylactic acid-sirolimus coating covering an outer surface of the pure zinc layer. The mass of iron in the stent body was 4 mg; the zinc layer had a mass of 250 μg; the polylactic acid in the coating was poly-racemic lactic acid with a molecular weight of 200 kDa. The average thicknesses of the poly-racemic lactic acid coatings on the inner surface and the side surface were 4.5 μm. The mass ratio of the poly-racemic lactic acid to the sirolimus on the outer surface was 4: 1, the average thickness was 8.8 μm, and the thickness of the polylactic acid coating was converted to be 7.1 μm. The polylactic acid coating and the polylactic acid-sirolimus coating were prepared by the spraying method.

Comparative Example 7-1

An iron-based stent with a specification of 3.0 mm × 8 mm included a stent body made of an iron-based alloy, a pure zinc layer completely covering the surface of the stent body, a polylactic acid coating covering an inner surface and a side surface of the pure zinc layer, and a polylactic acid-sirolimus coating covering an outer surface of the pure zinc layer. The mass of iron in the stent body was 4 mg; the zinc layer had a mass of 250 μg; the polylactic acid in the coating was poly-racemic lactic acid with a molecular weight of 200 kDa. The average thicknesses of the poly-racemic lactic acid coatings on the inner surface and the side surface were 4.5 μm. The mass ratio of the poly-racemic lactic acid to the sirolimus on the outer surface was 4: 1, the average thickness was 6 μm, and the thickness of the polylactic acid coating was converted to be 4.8 μm. The polylactic acid coating and the polylactic acid-sirolimus coating were prepared by the spraying method.

Comparative Example 7-2

An iron-based stent with a specification of 3.0 mm × 8 mm included a stent body made of an iron-based alloy, a pure zinc layer completely covering the surface of the stent body, a polylactic acid coating covering an inner surface and a side surface of the pure zinc layer, and a polylactic acid-sirolimus coating covering an outer surface of the pure zinc layer. The mass of iron in the stent body was 4 mg; the zinc layer had a mass of 250 μg; the polylactic acid in the coating was poly-racemic lactic acid with a molecular weight of 200 kDa. The average thicknesses of the poly-racemic lactic acid coatings on the inner surface and the side surface were 4.5 μm. The mass ratio of the poly-racemic lactic acid to the sirolimus on the outer surface was 4: 1, the average thickness was 14.5 μm, and the thickness of the polylactic acid coating was converted to be 11.6 μm. The polylactic acid coating and the polylactic acid-sirolimus coating were prepared by the spraying method.

The iron-based stents of Example 7, Comparative Example 7-1, and Comparative Example 7-2 were respectively implanted into the iliac arteries of three rabbits and were taken out after 1 month. The zinc corrosion rates were measured to be 51%, 61%, and 63%, respectively. The corrosion rate of zinc in the iron-based stent of Example 7 is the smallest.

Example 8

An iron-based stent with a specification of 3.0 mm × 8 mm included a stent body made of an iron-based alloy and a polylactic acid coating completely covering the surface of the stent matrix. The mass of iron in the stent body was 4 mg; the zinc layer had a mass of 250 μg; the polylactic acid in the coating was poly-racemic lactic acid with a molecular weight of 200 kDa. The average thickness of the poly-racemic lactic acid coating on an inner surface was 5.1 μm, and average thicknesses of the polylactic acid coatings on an outer surface and a side surface were 7.1 μm. The polylactic acid coating was prepared by the spraying method.

Comparative Example 8-1

An iron-based stent with a specification of 3.0 mm × 8 mm included a stent body made of an iron-based alloy and a polylactic acid coating completely covering the surface of the stent matrix. The mass of iron in the stent body was 4 mg; the zinc layer had a mass of 250 μg; the polylactic acid in the coating was poly-racemic lactic acid with a molecular weight of 200 kDa. The average thickness of the poly-racemic lactic acid coating on an inner surface was 5.1 μm, and the average thicknesses of the polylactic acid coatings on an outer surface and a side surface were 6.3 μm. The polylactic acid coating was prepared by the spraying method.

Comparative Example 8-2

An iron-based stent with a specification of 3.0 mm × 8 mm included a stent body made of an iron-based alloy and a polylactic acid coating completely covering the surface of the stent matrix. The mass of iron in the stent body was 4 mg; the zinc layer had a mass of 250 μg; the polylactic acid in the coating was poly-racemic lactic acid with a molecular weight of 200 kDa. The average thickness of the poly-racemic lactic acid coating on an inner surface was 5.1 μm, and the average thicknesses of the polylactic acid coatings on an outer surface and a side surface were 14.8 μm. The polylactic acid coating was prepared by the spraying method.

The iron-based stents of Example 8, Comparative Example 8-1, and Comparative Example 8-2 were respetively implanted into the iliac arteries of three rabbits and were taken out after 1 month. The zinc corrosion rates were measured to be 48%, 54%, and 66%, respectively. The corrosion rate of zinc in the iron-based stent of Example 8 is the smallest.

Example 9

An iron-based stent with a specification of 3.0 mm × 8 mm included a stent body made of an iron-based alloy and a polylactic acid coating completely covering the surface of the stent matrix. The mass of iron in the stent body was 4 mg; the zinc layer had a mass of 250 μg; the polylactic acid in the coating was poly-racemic lactic acid with a molecular weight of 200 kDa. The average thickness of the poly-racemic lactic acid coating on an inner surface was 7.1 μm, and the average thicknesses of the polylactic acid coatings on an outer surface and a side surface were 11.1 μm. The polylactic acid coating was prepared by the spraying method.

Comparative Example 9-1

An iron-based stent with a specification of 3.0 mm × 8 mm included a stent body made of an iron-based alloy and a polylactic acid coating completely covering the surface of the stent matrix. The mass of iron in the stent body was 4 mg; the zinc layer had a mass of 250 μg; the polylactic acid in the coating was poly-racemic lactic acid with a molecular weight of 200 kDa. The average thickness of the poly-racemic lactic acid coating on an inner surface was 7.1 μm, and the average thicknesses of the polylactic acid coatings on an outer surface and a side surface were 6.7 μm. The polylactic acid coating was prepared by the spraying method.

Example 9-2

An iron-based stent with a specification of 3.0 mm × 8 mm included a stent body made of an iron-based alloy and a polylactic acid coating completely covering the surface of the stent matrix. The mass of iron in the stent body was 4 mg; the zinc layer had a mass of 250 μg; polylactic acid in the coating was poly-racemic lactic acid with a molecular weight of 200 kDa. The average thickness of the poly-racemic lactic acid coating on an inner surface was 7.1 μm, and the average thicknesses of the polylactic acid coatings on an outer surface and a side surface were 16.6 μm. The polylactic acid coating was prepared by the spraying method.

The iron-based stents of Example 9, Example 9-1, and Example 9-2 were respectively implanted into the iliac arteries of three rabbits and were taken out after 1 month. The zinc corrosion rates were measured to be 44%, 64%, and 62%, respectively. The corrosion rate of zinc in the iron-based stent of Example 9 is the smallest.

Example 10

An iron-based stent with a specification of 3.0 mm × 8 mm included a stent body made of an iron-based alloy and a polylactic acid coating completely covering the surface of the stent matrix. The mass of iron in the stent body was 4 mg; the zinc layer had a mass of 250 μg; polylactic acid in the coating was poly-racemic lactic acid with a molecular weight of 100 kDa. The average thicknesses of the polylactic acid coatings on an inner surface, an outer surface, and a side surface were all 7.3 μm. The polylactic acid coating was prepared by the spraying method.

Comparative Example 10-1

An iron-based stent with a specification of 3.0 mm $\times$ 8 mm included a stent body made of an iron-based alloy and a polylactic acid coating completely covering the surface of the stent matrix. The mass of iron in the stent body was 4 mg; the zinc layer had a mass of 250 $\mu$g; the polylactic acid in the coating was poly-racemic lactic acid with a molecular weight of 100 kDa. The average thicknesses of the polylactic acid coatings on an inner surface, an outer surface, and a side surface were all 4 $\mu$m. The polylactic acid coating was prepared by the spraying method.

Comparative Example 10-2

An iron-based stent with a specification of 3.0 mm $\times$ 8 mm included a stent body made of an iron-based alloy and a polylactic acid coating completely covering the surface of the stent matrix. The mass of iron in the stent body was 4 mg; the zinc layer had a mass of 250 $\mu$g; the polylactic acid in the coating was poly-racemic lactic acid with a molecular weight of 100 kDa. The average thicknesses of the polylactic acid coatings on an inner surface, an outer surface, and a side surface were all 11 $\mu$m. The polylactic acid coating was prepared by the spraying method.

The iron-based stents of Example 10, Comparative Example 10-1, and Comparative Example 10-2 were respectively implanted into the iliac arteries of three rabbits and were taken out after 1 month. The zinc corrosion rates were measured to be 48%, 69%, and 55%, respectively. The corrosion rate of zinc in the iron-based stent of Example 10 is the smallest.

The protection scope of the patent of the present invention shall be subject to the appended claims.

**Claims**

1. A zinc-containing medical device, **characterized by** comprising a zinc-containing matrix and a polylactic acid coating arranged on the surface of the zinc-containing matrix, wherein the zinc-containing matrix has an outer surface, an inner surface, and a side surface; wherein when the zinc-containing medical device is implanted into the body, the inner surface is the surface in direct contact with body fluids, the outer surface is the surface in direct contact with the tissue walls, and the side surface is connected to the inner surface and outer surface;

the polylactic acid coating covers the outer surface, the inner surface, and the side surface;
the average thickness of the part located on the outer surface of the polylactic acid coating is $x_{outer}$;
the average thickness of the part located on the inner surface of the polylactic acid coating is $x_{inner}$;
the average thickness of the part located on the side surface of the polylactic acid coating is $x_{side}$, $x_{inner} \leq x_{outer}$, $x_{inner} \leq x_{side}$, and at least one of $x_{outer}$, $x_{inner}$, and $x_{side}$ satisfies the following formula:

wherein the polylactic acid coating has a thickness of $x$ that satisfies the following formula:

$$\left. \left( -b + \sqrt{b^2 - 4ac} \right) \middle/ 2a \right. - 2 \leq x \leq \left. \left( -b + \sqrt{b^2 - 4ac} \right) \middle/ 2a \right. + 2$$

wherein $x = x_{inner}$, $x_{side}$ or $x_{outer}$,
wherein, when the polylactic acid is poly-racemic lactic acid, a = 0.0336ln(Mn) - 0.1449, b = -0.472ln(Mn) + 2.1524, and c = 1.1604 ln(Mn)-5.7128;
when the polylactic acid is poly-L-lactic acid, a = -0.006ln(Mn) + 0.03441, b = 0.0648ln(Mn) -0.3662, and c = -0.162ln(Mn) + 0.7847;
Mn is the weight-average molecular weight of the polylactic acid and is in kDa; and $x$ is in $\mu$m;
wherein a material of the zinc-containing matrix is pure zinc or a zinc alloy or the zinc-containing matrix comprises a body and a zinc-containing layer attached to the body, wherein the material of the zinc-containing layer is pure zinc or a zinc alloy;
wherein, when the material of the zinc-containing matrix is pure zinc or the zinc alloy, the mass percentage of zinc in the zinc alloy is 50%-99.99% and, when the zinc-containing matrix comprises the body and the zinc-containing layer attached to the body and the material of the zinc-containing layer is the zinc alloy, the mass percentage of zinc in the zinc alloy is 50%-99.99%; and
when the zinc-containing matrix comprises the body and the zinc-containing layer attached to the body, the zinc-containing layer covers all surfaces of the body.

2. The zinc-containing medical device according to claim 1, **characterized in that** the polylactic acid is poly-racemic lactic acid with a weight-average molecular weight of 100-300 kDa;

the average thickness of the part located on the outer surface of the polylactic acid coating is 5.2-11.5 $\mu$m;
the average thickness of the part located on the inner surface of the polylactic acid coating is 5.2-11.5 $\mu$m; and
the average thickness of the part located on the side surface of the polylactic acid coating is 5.2-11.5 $\mu$m.

3. The zinc-containing medical device according to claim 1, **characterized in that** the polylactic acid is poly-racemic lactic acid with a weight-average molecular weight of 10-100 kDa;

the average thickness of the part located on the outer surface of the polylactic acid coating is 2-9 $\mu$m;
the average thickness of the part located on the inner surface of the polylactic acid coating is 2-9 $\mu$m; and
the average thickness of the part located on the side surface of the polylactic acid coating is 2-9 $\mu$m.

4. The zinc-containing medical device according to claim 1, **characterized in that** the polylactic acid is poly-racemic lactic acid with a weight-average molecular weight of 2-10 kDa;

the average thickness of the part located on the outer surface of the polylactic acid coating is 1.5-5.5 $\mu$m;
the average thickness of the part located on the inner surface of the polylactic acid coating is 1.5-5.5 $\mu$m; and
the average thickness of the part located on the side surface of the polylactic acid coating is 1.5-5.5 $\mu$m.

5. The zinc-containing medical device according to claim 1, **characterized in that** the polylactic acid is poly-L-lactic acid with a weight-average molecular weight of 200-300 kDa;

the average thickness of the part located on the outer surface of the polylactic acid coating is 9-22 $\mu$m;
the average thickness of the part located on the inner surface of the polylactic acid coating is 9-22 $\mu$m; and
the average thickness of the part located on the side surface of the polylactic acid coating is 9-22 $\mu$m.

6. The zinc-containing medical device according to claim 1, **characterized in that** the polylactic acid is poly-L-lactic acid with a weight-average molecular weight of 50-200 kDa;

the average thickness of the part located on the outer surface of the polylactic acid coating is 7-13 $\mu$m;
the average thickness of the part located on the inner surface of the polylactic acid coating is 7-13 $\mu$m; and
the average thickness of the part located on the side surface of the polylactic acid coating is 7-13 $\mu$m.

7. The zinc-containing medical device according to claim 1, **characterized in that** the polylactic acid coating contains an active drug.


**Patentansprüche**

1. Zinkhaltige medizinische Vorrichtung, **dadurch gekennzeichnet, dass** sie eine zinkhaltige Matrix und eine Polymilchsäurebeschichtung umfasst, die auf der Oberfläche der zinkhaltigen Matrix angeordnet ist, wobei die zinkhaltige Matrix eine Außenfläche, eine Innenfläche und eine Seitenfläche aufweist, wobei dann, wenn die zinkhaltige medizinische Vorrichtung in den Körper implantiert ist, die Innenfläche die in unmittelbarem Kontakt mit Körperflüssigkeiten stehende Fläche ist, die Außenfläche die in unmittelbarem Kontakt mit den Gewebewänden stehende Fläche ist und die Seitenfläche mit der Innenfläche und der Außenfläche verbunden ist,

die Polymilchsäurebeschichtung die Außenfläche, die Innenfläche und die Seitenfläche bedeckt,
die mittlere Dicke des an der Außenfläche befindlichen Teils der Polymilchsäurebeschichtung $x_{außen}$ ist,
die mittlere Dicke des an der Innenfläche befindlichen Teils der Polymilchsäurebeschichtung $x_{innen}$ ist,
die mittlere Dicke des an der Seitenfläche befindlichen Teils der Polymilchsäurebeschichtung $x_{Seite}$ ist, $x_{innen} \leq x_{außen}$ ist, $x_{innen} \leq x_{Seite}$ ist und $x_{außen}$ und/oder $x_{innen}$ und/oder $x_{Seite}$ die folgende Formel erfüllt bzw. erfüllen:

wobei die Polymilchsäurebeschichtung eine Dicke $x$ aufweist, die die folgende Formel erfüllt:

$$\left(-b + \sqrt{b^2 - 4ac}\right)\Big/2a - 2 \leq x \leq \left(-b + \sqrt{b^2 - 4ac}\right)\Big/2a + 2,$$

wobei $x = x_{innen}$, $x_{Seite}$ oder $x_{außen}$,

wobei dann, wenn die Polymilchsäure polyracemische Milchsäure ist, a = 0,0336In(Mn) - 0,1449, b = -0,472In(Mn) + 2,1524 und c = 1,1604 In(Mn) - 5,7128,

dann, wenn die Polymilchsäure Poly-L-Milchsäure ist, a = -0,006In(Mn) + 0,03441, b = 0,0648In(Mn) -0,3662 und c = -0,162In(Mn) + 0,7847,

Mn das gewichtsgemittelte Molekulargewicht der Polymilchsäure ist und in kDa angegeben ist und $x$ in $\mu$m angegeben ist,

wobei ein Material der zinkhaltigen Matrix reines Zink oder eine Zinklegierung ist oder die zinkhaltige Matrix einen Körper und eine am Körper angebrachte zinkhaltige Schicht umfasst, wobei das Material der zinkhaltigen Schicht reines Zink oder eine Zinklegierung ist,

wobei dann, wenn das Material der zinkhaltigen Matrix reines Zink oder die Zinklegierung ist, der Massenanteil an Zink in der Zinklegierung 50% - 99,99% beträgt, und wenn die zinkhaltige Matrix den Körper und die am Körper angebrachte zinkhaltige Schicht umfasst und das Material der zinkhaltigen Schicht die Zinklegierung ist, der Massenanteil an Zink in der Zinklegierung 50% - 99,99% beträgt, und

dann, wenn die zinkhaltige Matrix den Körper und die am Körper angebrachte zinkhaltige Schicht umfasst, die zinkhaltige Schicht alle Oberflächen des Körpers bedeckt.

2. Zinkhaltige medizinische Vorrichtung nach Anspruch 1, **dadurch gekennzeichnet, dass** die Polymilchsäure polyracemische Milchsäure mit einem gewichtsgemittelten Molekulargewicht von 100 - 300 kDa ist,

die mittlere Dicke des an der Außenfläche befindlichen Teils der Polymilchsäurebeschichtung 5,2 - 11,5 $\mu$m beträgt,

die mittlere Dicke des an der Innenfläche befindlichen Teils der Polymilchsäurebeschichtung 5,2 - 11,5 $\mu$m beträgt und

die mittlere Dicke des an der Seitenfläche befindlichen Teils der Polymilchsäurebeschichtung 5,2 - 11,5 $\mu$m beträgt.

3. Zinkhaltige medizinische Vorrichtung nach Anspruch 1, **dadurch gekennzeichnet, dass** die Polymilchsäure polyracemische Milchsäure mit einem gewichtsgemittelten Molekulargewicht von 10 - 100 kDa ist,

die mittlere Dicke des an der Außenfläche befindlichen Teils der Polymilchsäurebeschichtung 2 - 9 $\mu$m beträgt,

die mittlere Dicke des an der Innenfläche befindlichen Teils der Polymilchsäurebeschichtung 2 - 9 $\mu$m beträgt und

die mittlere Dicke des an der Seitenfläche befindlichen Teils der Polymilchsäurebeschichtung 2 - 9 $\mu$m beträgt.

4. Zinkhaltige medizinische Vorrichtung nach Anspruch 1, **dadurch gekennzeichnet, dass** die Polymilchsäure polyracemische Milchsäure mit einem gewichtsgemittelten Molekulargewicht von 2 - 10 kDa ist,

die mittlere Dicke des an der Außenfläche befindlichen Teils der Polymilchsäurebeschichtung 1,5 - 5,5 $\mu$m beträgt,

die mittlere Dicke des an der Innenfläche befindlichen Teils der Polymilchsäurebeschichtung 1,5 - 5,5 $\mu$m beträgt und

die mittlere Dicke des an der Seitenfläche befindlichen Teils der Polymilchsäurebeschichtung 1,5 - 5,5 $\mu$m beträgt.

5. Zinkhaltige medizinische Vorrichtung nach Anspruch 1, **dadurch gekennzeichnet, dass** die Polymilchsäure Poly-L-Milchsäure mit einem gewichtsgemittelten Molekulargewicht von 200 - 300 kDa ist,

die mittlere Dicke des an der Außenfläche befindlichen Teils der Polymilchsäurebeschichtung 9 - 22 $\mu$m beträgt,

die mittlere Dicke des an der Innenfläche befindlichen Teils der Polymilchsäurebeschichtung 9 - 22 $\mu$m beträgt und

die mittlere Dicke des an der Seitenfläche befindlichen Teils der Polymilchsäurebeschichtung 9 - 22 $\mu$m beträgt.

6. Zinkhaltige medizinische Vorrichtung nach Anspruch 1, **dadurch gekennzeichnet, dass** die Polymilchsäure Poly-L-Milchsäure mit einem gewichtsgemittelten Molekulargewicht von 50 - 200 kDa ist,

die mittlere Dicke des an der Außenfläche befindlichen Teils der Polymilchsäurebeschichtung 7 - 13 μm beträgt,

die mittlere Dicke des an der Innenfläche befindlichen Teils der Polymilchsäurebeschichtung 7 - 13 μm beträgt und

die mittlere Dicke des an der Seitenfläche befindlichen Teils der Polymilchsäurebeschichtung 7 - 13 μm beträgt.

**7.** Zinkhaltige medizinische Vorrichtung nach Anspruch 1, **dadurch gekennzeichnet, dass** die Polymilchsäurebeschichtung einen Arzneimittelwirkstoff enthält.

## Revendications

**1.** Dispositif médical contenant du zinc, **caractérisé en ce qu'**il comprend une matrice contenant du zinc et un revêtement en acide polylactique disposé sur la surface de la matrice contenant du zinc, la matrice contenant du zinc présentant une surface extérieure, une surface intérieure et une surface latérale, la surface intérieure, lorsque le dispositif médical contenant du zinc est implanté dans le corps, étant la surface en contact direct avec les fluides corporels, la surface extérieure étant la surface en contact direct avec les parois tissulaires, et la surface latérale étant reliée à la surface intérieure et à la surface extérieure ;

le revêtement en acide polylactique recouvrant la surface extérieure, la surface intérieure et la surface latérale ;
l'épaisseur moyenne de la partie située sur la surface extérieure du revêtement en acide polylactique étant $x_{\text{extérieure}}$ ;
l'épaisseur moyenne de la partie située sur la surface intérieure du revêtement en acide polylactique étant $x_{\text{intérieure}}$ ;
l'épaisseur moyenne de la partie située sur la surface latérale du revêtement en acide polylactique étant $x_{\text{latérale}}$, $x_{\text{intérieure}} \leq x_{\text{extérieure}}$, $x_{\text{intérieure}} \leq x_{\text{latérale}}$, et $x_{\text{extérieure}}$ et/ou $x_{\text{intérieure}}$ et/ou $x_{\text{latérale}}$ satisfaisant à la formule suivante :

le revêtement en acide polylactique présentant une épaisseur $x$ qui satisfait à la formule suivante :

$$\left.\left(-b + \sqrt{b^2 - 4ac}\right)\middle/ 2a\right. - 2 \leq x \leq \left.\left(-b + \sqrt{b^2 - 4ac}\right)\middle/ 2a\right. + 2$$

avec $x = x_{\text{intérieure}}$, $x_{\text{latérale}}$ ou $x_{\text{extérieure}}$ ;
lorsque l'acide polylactique est de l'acide lactique polyracémique, a = 0,0336ln(Mn) - 0,1449, b = -0,472ln(Mn) + 2,1524, et c = 1,1604ln(Mn) - 5,7128 ;
lorsque l'acide polylactique est de l'acide poly-L-lactique, a = -0,006ln(Mn) + 0,03441, b = 0,0648ln(Mn) - 0,3662, et c = -0,162ln(Mn) + 0,7847 ;
Mn étant le poids moléculaire moyen en poids de l'acide polylactique et étant exprimé en kDa, et $x$ étant exprimé en μm ;
un matériau de la matrice contenant du zinc étant du zinc pur ou un alliage de zinc, ou la matrice contenant du zinc comprenant un corps et une couche contenant du zinc fixée au corps, le matériau de la couche contenant du zinc étant du zinc pur ou un alliage de zinc ;
lorsque le matériau de la matrice contenant du zinc est du zinc pur ou l'alliage de zinc, le pourcentage massique de zinc dans l'alliage de zinc étant compris entre 50 % et 99,99 %, et lorsque la matrice contenant du zinc comprend le corps et la couche contenant du zinc fixée au corps et le matériau de la couche contenant du zinc est l'alliage de zinc, le pourcentage massique de zinc dans l'alliage de zinc étant compris entre 50 % et 99,99 % ; et
lorsque la matrice contenant du zinc comprend le corps et la couche contenant du zinc fixée au corps, la couche contenant du zinc recouvrant toutes les surfaces du corps.

**2.** Dispositif médical contenant du zinc selon la revendication 1, **caractérisé en ce que** l'acide polylactique est de l'acide lactique polyracémique présentant un poids moléculaire moyen en poids de 100 à 300 kDa ;

l'épaisseur moyenne de la partie située sur la surface extérieure du revêtement en acide polylactique étant comprise entre 5,2 et 11,5 μm ;
l'épaisseur moyenne de la partie située sur la surface intérieure du revêtement en acide polylactique étant comprise entre 5,2 et 11,5 μm ; et

l'épaisseur moyenne de la partie située sur la surface latérale du revêtement en acide polylactique étant comprise entre 5,2 et 11,5 μm.

3. Dispositif médical contenant du zinc selon la revendication 1, **caractérisé en ce que** l'acide polylactique est de l'acide lactique polyracémique présentant un poids moléculaire moyen en poids de 10 à 100 kDa ;

l'épaisseur moyenne de la partie située sur la surface extérieure du revêtement en acide polylactique étant comprise entre 2 et 9 μm ;
l'épaisseur moyenne de la partie située sur la surface intérieure du revêtement en acide polylactique étant comprise entre 2 et 9 μm ; et
l'épaisseur moyenne de la partie située sur la surface latérale du revêtement en acide polylactique étant comprise entre 2 et 9 μm.

4. Dispositif médical contenant du zinc selon la revendication 1, **caractérisé en ce que** l'acide polylactique est de l'acide lactique polyracémique présentant un poids moléculaire moyen en poids de 2 à 10 kDa ;

l'épaisseur moyenne de la partie située sur la surface extérieure du revêtement en acide polylactique étant comprise entre 1,5 et 5,5 μm ;
l'épaisseur moyenne de la partie située sur la surface intérieure du revêtement en acide polylactique étant comprise entre 1,5 et 5,5 μm ; et
l'épaisseur moyenne de la partie située sur la surface latérale du revêtement en acide polylactique étant comprise entre 1,5 et 5,5 μm.

5. Dispositif médical contenant du zinc selon la revendication 1, **caractérisé en ce que** l'acide polylactique est de l'acide poly-L-lactique présentant un poids moléculaire moyen en poids de 200 à 300 kDa ;

l'épaisseur moyenne de la partie située sur la surface extérieure du revêtement en acide polylactique étant comprise entre 9 et 22 μm ;
l'épaisseur moyenne de la partie située sur la surface intérieure du revêtement en acide polylactique étant comprise entre 9 et 22 μm ; et
l'épaisseur moyenne de la partie située sur la surface latérale du revêtement en acide polylactique étant comprise entre 9 et 22 μm.

6. Dispositif médical contenant du zinc selon la revendication 1, **caractérisé en ce que** l'acide polylactique est de l'acide poly-L-lactique présentant un poids moléculaire moyen en poids de 50 à 200 kDa ;

l'épaisseur moyenne de la partie située sur la surface extérieure du revêtement en acide polylactique étant comprise entre 7 et 13 μm ;
l'épaisseur moyenne de la partie située sur la surface intérieure du revêtement en acide polylactique étant comprise entre 7 et 13 μm ; et
l'épaisseur moyenne de la partie située sur la surface latérale du revêtement en acide polylactique étant comprise entre 7 et 13 μm.

7. Dispositif médical contenant du zinc selon la revendication 1, **caractérisé en ce que** le revêtement en acide polylactique contient un médicament actif.

Fig. 1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016167460 A1
- CN 104857570 A
- CN 106474545 A